(19) Europäisches Patentamt
European Patent Office
Office européen des brevets

(11) Veröffentlichungsnummer: **0 481 366 A1**

# (12) EUROPÄISCHE PATENTANMELDUNG

(21) Anmeldenummer: **91117334.2**

(51) Int. Cl.[5]: **C07D 277/78**, C07D 277/36

(22) Anmeldetag: **11.10.91**

(30) Priorität: **15.10.90 DE 4032680**

(43) Veröffentlichungstag der Anmeldung:
**22.04.92 Patentblatt 92/17**

(84) Benannte Vertragsstaaten:
**BE DE ES FR GB IT**

(71) Anmelder: **Akzo N.V.**
**Postbus 9300 Velperweg 76**
**NL-6800 SB Arnhem(NL)**

(72) Erfinder: **Bergfeld, Manfred Josef, Dr.**
**August-Pfefferstrasse 6**
**W-8765 Erlenbach(DE)**
Erfinder: **Eisenhuth, Ludwig, Dr.**
**Lauterhofstrasse 44**
**W-8753 Obernburg(DE)**

(74) Vertreter: **Fett, Günter et al**
**Akzo Patente GmbH Kasinostrasse 19 - 23**
**W-5600 Wuppertal 1(DE)**

## (54) Verfahren zur Herstellung von 2-Aminodithiothiazolen und 2-Aminotrithiothiazolen.

(57) Das Verfahren zur Herstellung von 2-(Aminodithio)-thiazolen und 2-(Aminotrithio)-thiazolen durch Umsetzung einer Mischung von einem 2-Mercaptothiazol oder einem Dithiazolyl-2,2'-disulfid mit einem gesättigten sekundären heterocyclischen Amin und Schwefel in einem ein inertes organisches Lösungsmittel enthaltenden Reaktionsmedium in Gegenwart eines Oxidationsmittels ist dadurch gekennzeichnet, daß die Umsetzung in Gegenwart von Ammoniak und eines Kupfer, eine Kupferverbindung oder eine Cer-Verbindung enthaltenden Katalysators durchgeführt wird und daß das Oxidationsmittel molekularer Sauerstoff oder ein diesen Sauerstoff enthaltendes Gas ist. Insbesondere das technisch als Vulkanisationsbeschleuniger interessante 2-(4-Morpholinodithio)-benzthiazol läßt sich mit hoher Produktausbeute und praktisch ohne Nebenprodukte mit dem billigeren und leichter handhabbaren Oxidationsmittel herstellen.

EP 0 481 366 A1

Die vorliegende Erfindung betrifft ein Verfahren zur Herstellung von 2-(Aminodithio)-thiazolen und 2-(Aminotrithio)-thiazolen durch Umsetzung eines cyclischen sekundären Amins, eines 2-Mercapto-thiazols und von Schwefel in Gegenwart eines Oxidationsmittels in einem inerten organischen Lösungsmittel.

Ein typischer Vertreter der Verbindungen aus der Gruppe der 2-Aminodithiothiazole ist das 2-(4-Morpholinodithio)-benzthiazol, welches in großen Mengen Einsatz als Vulkanisationsbeschleuniger und Schwefelspender findet. Es wurden zahlreiche Verfahren zur Herstellung von 2-(4-Morpholinodithio)-benzthiazol beschrieben. Man kann sie nach den jeweils eingesetzten Ausgangsverbindungen wie folgt zusammenfassen (in Klammern Vorveröffentlichung):
aus Morpholinsulfiden und 2-Mercaptobenzthiazol oder Dibenzthiazolyl-2,2'-disulfid (US-PS 4,489,754), aus 2-Mercaptobenzthiazol oder Dibenzthiazolyl-2,2'-disulfid, 4-Morpholin und Dischwefeldichlorid (US-PS 3,070,593), aus 2-Mercaptobenzthiazol, 4-Morpholin, Chlor und Dischwefeldichlorid (US-PS 2,983,726), aus 2-Mercaptobenzthiazol oder Dibenzthiazolyl-2,2'-disulfid, 4-Morpholin, Schwefel und Oxidationsmitteln wie Natriumhypochlorit (DE-A 22 38 516), aus Morpholinobenzthiazol und Schwefel (DE-A 11 34 677), sowie aus Sulfenamiden, 4-Morpholin und Schwefel (US-PS 3,969,350). Von diesem Verfahren bieten sich diejenigen für eine technische Ausführung an, welche von den preiswerten und gut verfügbaren Rohstoffen 2-Mercaptobenzthiazol, 4-Morpholin und Schwefel ausgehen, beschrieben in DE-A 22 38 516 wie auch in DE-A 21 64 480 und US-PS 3,281,418. Gemäß Inhalt dieser drei Druckschriften müssen aber immer noch wenig preisgünstige Oxidationsmittel in hohem stöchiometrischem Überschuß eingesetzt werden, insbesondere solche, die merklich in Wasser löslich sind, um sie in wäßriger Lösung einzusetzen. Beispielsweise sollen Ammonium- oder Kalium-peroxidisulfat, Wasserstoffperoxid, Kaliumpermanganat und Natrium- oder Calciumhypochlorit verwendet werden, wobei dem Natriumhypochlorit in allen Fällen der Vorzug gegeben wird. Nachteilig wirkt sich bei Verwendung der meisten dieser Oxidationsmittel aus, daß anorganische Salze als Beiprodukt gebildet werden, beispielsweise Natriumchlorid im Falle des bevorzugten Natriumhypochlorits, welche die Abwässer belasten bzw. abgetrennt werden müssen. Auch müssen hohe stöchiometrische Überschüsse nicht nur des Oxidationsmittels, sondern auch der übrigen Reaktionspartner in diesen Verfahren angewendet werden.

Einen Hinweis, wie das Verfahren zur Herstellung von 2-(4-Morpholinodithio)-benzthiazol aus 2-Mercaptobenzthiazol, 4-Morpholin, Schwefel und Oxidatonsmittel ökonomischer gestaltet werden kann, gibt die genannte Druckschrift DE-A 22 38 516. Hiernach soll das 2-Mercaptobenzthiazol direkt in dem Zustand eingesetzt werden, in dem es aus der Herstellung von 2-Mercaptobenzthiazol anfällt, nämlich wasserfeucht, wodurch dessen Trocknungsstufe entfällt.

Es ist nun Aufgabe der vorliegenden Erfindung, das Verfahren zur Herstellung von 2-Aminodithiothiazolen aus 2-Mercaptothiazolen, sekundären cyclischen Aminen, Schwefel und Oxidationsmittel ökonomischer und mit geringerer Abwasserbelastung zu gestalten, und zwar durch Ersatz der bisher eingesetzten Oxidationsmittel durch ein preisgünstigeres und einfacher handhabbares Produkt.

Diese Aufgabe wird gelöst durch ein Verfahren zur Herstellung von 2-(Aminodithio)-thiazolen und/oder 2-(Aminotrithio)-thiazolen der allgemeinen Formel (I)

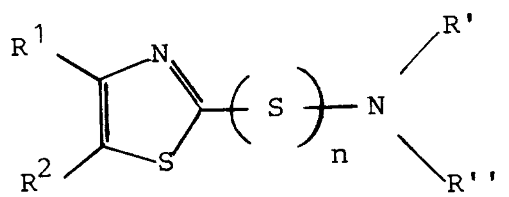

(I)

in der n im wesentlichen 2 oder 3 ist, in der $R^1$ und $R^2$ gleich oder verschieden sein können und jeweils für ein Wasserstoffatom, ein Halogenatom, eine Nitrogruppe, eine Hydroxylgruppe oder für einen gegebenenfalls ein- oder mehrfach substituierten organischen Rest wie einen Alkyl- oder Alkoxylrest mit 1 bis 6 Kohlenstoffatomen oder einen Cycloalkyl- oder Arylrest mit 6 bis 12 Kohlenstoffatomen stehen, wobei die Substituenten jeweils ein Halogenatom, eine Nitrogruppe, eine Hydroxylgruppe oder einen Alkyl- oder Alkoxyrest mit 1 bis 5 Kohlenstoffatomen sein können oder in der $R^1$ und $R^2$ gemeinsam den Rest (II)

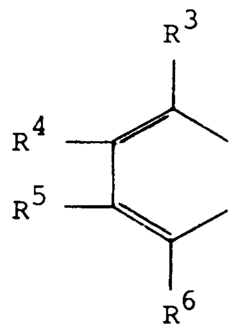

oder

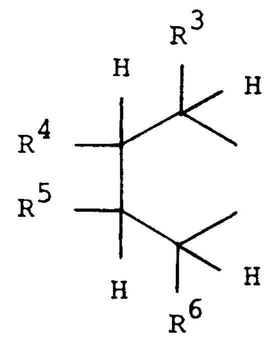

(II)

bilden, wobei $R^3$, $R^4$, $R^5$ und $R^6$ gleich oder verschieden sein können und jeweils dieselbe Bedeutung haben wie $R^1$ und $R^2$, nicht jedoch den Rest (II) bilden, und in der R' und R'' zusammen mit dem Aminstickstoff einen aliphatisch gesättigten heterocyclischen Ring bilden, der mindestens ein weiteres Heteroatom enthalten kann, wobei mit Piperazin als Basis des heterocyclischen Rings das weitere Stickstoffatom ebenfalls den 2-Thiazolyldithio- bzw. 2-Thiazolyltrithio-Rest tragen kann, durch Umsetzung einer Mischung eines 2-Mercaptothiazols der allgemeinen Formel (III) oder Dithiazolyl-2,2'-disulfids der allgemeinen Formel (IV)

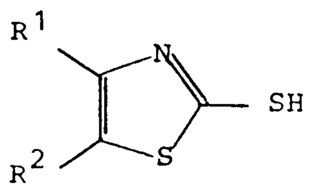

(III)

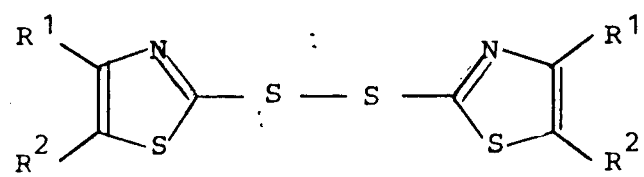

(IV)

in der $R_1$ und $R_2$ die oben angegebene Bedeutung haben, eines gesättigten sekundären, heterocyclischen Amins der allgemeinen Formel (V)

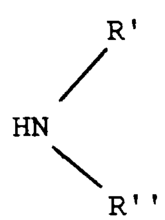

(V)

mit der oben angegebenen Bedeutung für R' und R'' und Schwefel in einem ein inertes organisches Lösungsmittel enthaltenden Reaktionsmedium in Gegenwart eines Oxidationsmittels, dadurch gekennzeichnet, daß die Umsetzung in Gegenwart von Ammoniak und eines Kupfer, eine Kupferverbindung oder eine Cer-Verbindung enthaltenden Katalysators durchgeführt wird und daß das Oxidationsmittel molekularer Sauerstoff oder ein diesen Sauerstoff enthaltendes Gas ist.

Das mit diesem neuen Verfahren erreichbare Ergebnis ist nur mit cyclischen Aminen (V), nicht jedoch mit acyclischen, aliphatischen Aminen ausführbar; entsprechende Produkte mit cyclischen Aminen sind bislang in der Literatur nicht bekannt. Durch Weglassen des Schwefels unter sonst erfindungsgemäßen Verfahrensbedingungen (also Umsetzung nur von Mercaptothiazol und cyclischem Amin in Gegenwart von Sauerstoff) werden andererseits nur geringe Mengen an Cycloaminomonothiothiazol erhalten (s. Versuch V2 in Tabelle 1). Dies ist überraschend wegen des Inhalts von DE-A 33 25 724, welche die Herstellung von Aminomonothiothiazolen (dort als "Sulfenamide" bezeichnet) aus 2-Mercaptobenzthiazol und Aminen in

Gegenwart von Sauerstoff beschreibt. Wegen DE-A 33 25 724 war nämlich anzunehmen, daß intermediär ein solches Monothiothiazol gebildet wird. Da dies nicht der Fall ist, muß also ein anderer Reaktionsmechanismus im erfindungsgemäßen Verfahren vorliegen.

Die Substituenten $R^1$ bis $R^6$ der allgemeinen Formeln I, II, III und IV sind vorzugsweise ein Chlor- oder Bromatom, eine Hydroxylgruppe, eine Nitrogruppe, ein geradkettiger oder verzweigter Alkylrest mit 1 - 4 Kohlenstoffatomen wie Methyl-, Ethyl-, Propyl-, Isopropyl-, Butyl- oder tert. Butyl-, ein Alkoxyrest mit 1 - 4 Kohlenstoffatomen wie Methoxy-, Ethoxy-, Propoxy- oder Butoxy- oder ein Phenyl-, Tolyl-, Ethylphenyl-, Nitrophenyl-, Chlorphenyl-, Bromphenyl- oder Naphthylrest.

Das erfindungsgemäße Verfahren ist insbesondere für die Herstellung von 2-(Aminodithio)-thiazolen und 2-(Aminotrithio)-thiazolen aus 2-Mercaptobenzthiazol bzw. Dibenzthiazolyl-2,2'-disulfid, der wichtigsten Vertreter der 2-Mercaptothiazole bzw. Dithiazolyl-2,2'-disulfide, geeignet. Beispiele für andere 2-Mercaptothiazole, welche sich als Startverbindungen für die erfindungsgemäße Herstellung von 2-Aminodithiothiazolen der allgemeinen Formel (I) eignen, sind die folgenden Verbindungen:

2-Mercaptothiazol
2-Mercapto-4-methylthiazol
2-Mercapto-4-ethylthiazol
2-Mercapto-4-n-propylthiazol
2-Mercapto-4-n-butylthiazol
2-Mercapto-4,5-dimethylthiazol
2-Mercapto-4,5-diethylthiazol
2-Mercapto-4,5-di-n-propylthiazol
2-Mercapto-4,5-di-n-butylthiazol
2-Mercapto-4-phenylthiazol
2-Mercapto-4-phenyl-5-methylthiazol
2-Mercapto-4-phenyl-5-chlor-thiazol
2-Mercapto-4-m-chlorphenylthiazol
2-Mercapto-4-p-bromphenylthiazol
2-Mercapto-4-m-nitrophenylthiazol
2-Mercapto-4-methylbenzthiazol
2-Mercapto-5-methylbenzthiazol
2-Mercapto-6-methylbenzthiazol
2-Mercapto-4,5-dimethylbenzthiazol
2-Mercapto-4-phenylbenzthiazol
2-Mercapto-6-phenylbenzthiazol
2-Mercapto-4-methoxybenzthiazol
2-Mercapto-6-methoxybenzthiazol
2-Mercapto-5,6-dimethoxybenzthiazol
2-Mercapto-6-methoxy-4-nitrobenzthiazol
2-Mercapto-6-ethoxybenzthiazol
2-Mercapto-4-chlorbenzthiazol
2-Mercapto-5-chlorbenzthiazol
2-Mercapto-6-chlorbenzthiazol
2-Mercapto-7-chlorbenzthiazol
2-Mercapto-5-chlor-6-methoxybenzthiazol
2-Mercapto-5-chlor-4-nitrobenzthiazol
2-Mercapto-5-chlor-6-nitrobenzthiazol
2-Mercapto-4,5-dichlorbenzthiazol
2-Mercapto-4,7-dichlorbenzthiazol
2-Mercapto-5-nitrobenzthiazol
2-Mercapto-6-nitrobenzthiazol
2-Mercapto-6-hydroxybenzthiazol
2-Mercapto-tetrahydrobenzthiazol
2-Mercapto-naphthothiazol

Beispiele für andere Dithiazolyl-2,2'-disulfide als Startverbindungen im erfindungsgemäßen Verfahren sind die folgenden Verbindungen:

Bis-(6-methylbenzthiazolyl)-2,2'-disulfid
Bis-(4-methylbenzthiazolyl)-2,2'-disulfid
Bis-(4-methoxybenzthiazolyl)-2,2'-disulfid

Bis-(6-äthoxybenzthiazolyl)-2,2'-disulfid
Bis-(6-chlorbenzthiazolyl)-2,2'-disulfid
Bis-(5-chlor-4-nitrobenzthiazolyl)-2,2'-disulfid
Bis-(3-chlor-6-nitrobenzthiazolyl)-2,2'-disulfid
Bis-(6-nitrobenzthiazolyl)-2,2'-disulfid
Bis-(tetrahydrobenzthiazolyl)-2,2'-disulfid

Dithiazolyl-2,2'-disulfide haben gegenüber 2-Mercaptothiazolen als startverbindungen den Vorteil, daß die chemische Umsetzung wesentlich schneller abläuft (s. Versuchsbeispiel 5), da zur Umsetzung des Dithiazolyl-2,2'-disulfids nur halb so viel Sauerstoff benötigt wird wie für diejenige des 2-Mercaptothiazols.

Die Substituenten R' und R'' der allgemeinen Formeln (I) und (V) bilden erfindungsgemäß zusammen mit dem Aminstickstoff einen gesättigten heterocyclischen Ring, der mindestens ein weiteres Heteroatom noch enthalten kann, wobei unter einem Heteroatom insbesondere ein Sauerstoff-, Schwefel- und Stickstoffatom zu verstehen ist. R' und R'' bilden also eine Polymethylenbrücke bzw. eine Polymethylenbrücke mit mindestens einer insbesondere durch -O-, -S- und -NR- mit R = H, $CH_3$, $C_2H_5$, $C_3H_7$, $C_4H_9$, $CH_2$-$CH_2$-OH ersetzten Methylen-Gruppe. Im Falle einer Gruppe -NH- anstelle einer Methylengruppe, also bei Einsatz eines Piperazins als Startverbindung, kann auch eine mit dem 2-Thiazolyldithio- bzw. 2-Thiazolyltrithio-Rest gemäß Formel (I) zweifach N,N'-substituierte Verbindung gebildet werden, vorausgesetzt, daß entsprechend stöchiometrische Mengen, d.h. doppelte Mengen an Mercaptothiazol bzw. Thiazolyl-2,2'-disulfid eingesetzt werden. Der heterocyclische Ring ist bevorzugt 5-, 6- und 7-gliedrig und kann ein oder mehrere, bevorzugt ein oder zwei inerte Substituenten aufweisen, beispielsweise Alkylgruppen mit 1 bis 4 C-Atomen, bevorzugt Methyl- und Ethylgruppen, oder Alkylengruppen, bevorzugt eine Trimethylen- oder Tetramethylengruppe, welche mit 2 benachbarten C-Atomen des heterocyclischen Ringes einen weiteren 5- oder 6-gliedrigen Ring bilden.

Bevorzugte sekundäre heterocyclische Amine der allgemeinen Formel (V) als Startverbindungen im erfindungsgemäßen Verfahren sind unsubstituiertes oder mit Alkylgruppen mit 1 bis 4 C-Atomen substituiertes Pyrrolidin, Pyrazolidin, Imidazolidin, Oxazolidin, Thiazolidin und besonders bevorzugt Piperidin, Piperazin, Cyclohexamethylenimin und Morpholin, insbesondere 4-Morpholin. Beispiele für substituierte sekundäre heterocyclische Amine sind 2- oder 4-Methylpiperidin, N-Methylpiperazin, 2,6-Dimethylmorpholin und 3,4-Dimethylmorpholin.

2-Mercaptothiazol bzw. Dithiazolyl-2,2'-disulfid, heterocyclisches Amin und Schwefel können im erfindungsgemäßen Verfahren in stöchiometrischen Mengen oder die Startverbindung (V) in geringem stöchiometrischen Unterschuß oder in stöchiometrischem überschuß bis zu 100 Mol-% gegenüber den übrigen Startverbindungen zur Anwendung kommen. Bevorzugt werden 0.9 bis 2 Mol und besonders bevorzugt 0.9 bis 1.5 Mol Startverbindung (V) je 1 Mol 2-Mercaptothiazol bzw. Äquivalent Dithazolyl-2,2'-disulfid eingesetzt (Startverbindungen (III) bzw. (IV)). Fällen, abhängig von den Reaktionsbedingungen und vom verwendeten cyclischen Amin, werden mit geringen Aminüberschüssen von 0 bis 0.1 Mol gute Ergebnisse erzielt. Es können selbstverständlich auch höhere stöchiometrische Überschüsse an Startverbindung (V) angewandt werden, wie sie beispielsweise in den Verfahren des Standes der Technik üblich sind. Erfindungsgemäß ist unter "stöchiometrischem Überschuß" der Anteil des jeweiligen Reaktionspartners zu verstehen, der über die zur Einhaltung der exakten Stöchiometrie benötigten Mengen hinausgeht.

Bezüglich des Schwefels ist erfindungsgemäß von zweierlei stöchiometrischen Mengen auszugehen: Zur Herstellung der Dithiothiazole wird 1 Äquivalent und zur Herstellung der Trithiothiazole werden 2 Äquivalente Schwefel je Äquivalent 2-Mercaptobenzthiazol bzw. Dithiazolyl-2,2'-disulfid benötigt; Schwefel-Mengen im Bereich zwischen 1 und 2 Äquivalent führen entsprechend zu Gemischen von Dithiazol und Trithiazol. 3 Äquivalente Schwefel führen allerdings nicht zu einer entsprechenden Tetrathiazol-Verbindung; es resultiert dann lediglich ein Gemisch von Trithiothiazol und Schwefel.

Der Anteil des Oxidationsmittels "molekularer Sauerstoff" bzw. "molekularen Sauerstoff enthaltendes Gas" wird durch die Sauerstoffdrücke bzw. Sauerstoffpartialdrücke bestimmt. Sie betragen erfindungsgemäß bevorzugt höchstens $10^6$ Pa Überdruck aus ökonomischen und sicherheitstechnischen Gründen und mindestens $10^4$ Pa. Die Reaktionsgeschwindigkeit steigt mit steigendem Sauerstoffdruck.

Als Katalysator wird beim erfindungsgemäßen Verfahren metallisches Kupfer, eine Kupferverbindung oder eine Cer-Verbindung, jeweils in Gegenwart von Ammoniak, eingesetzt. Das metallische Kupfer kommt vorzugsweise als Kupferpulver zur Anwendung. Als Kupferverbindung kommen alle ein- oder zweiwertigen anorganischen, organischen, einfachen oder komplexen Kupfersalze in Betracht. Beispiele für geeignete einwertige Kupfersalze sind Kupfer-(I)-chlorid, -bromid und -jodid, Additionsverbindungen dieser Kupfer-(I)-halogenide mit Kohlenmonoxid, komplexe Kupfer(I)-salze wie die Alkalichlorcuprate, komplexe Ammoniakate des Kupfer-(I)-cyanids, z.B. Cyanocuprate wie Kalium-tricyanocuprat(I), Doppelsalze mit Kupfer(I)-rhodanid, Kupfer-(I)-acetat, Kupfer-(I)-sulfit und komplexe Doppelsulfide aus Kupfer(I)-sulfid und Alkalipolysulfiden.

Beispiele geeigneter Kupfer-(II)-salze sind Kupfer-(II)-chlorid, -bromid, -sulfid, -sulfat, -nitrat, -nitrit, -rhodanid, -cyanid, Cu(II)-Salze von Carbonsäuren wie Kupfer(II)-acetat sowie die komplexen Ammoniakate von Kupfer-(II)-salzen. Auch Kupfer-(I)-oxid ist sehr gut als Katalysator geeignet. Als Cer-Verbindungen kommen alle 3- und 4-wertigen Cer-Verbindungen in Betracht, wie beispielsweise Cer-(III)-nitrat.

Bevorzugt werden als Katalysator Kupferpulver,
Kupfer-(I)-chlorid, Kupfer-(II)-acetat,
Kupfer-(II)-sulfat, Kupfer-(II)-oleat,
Kupfer-(II)-acetylacetonat, Kupfer-(II)-sulfid oder
Kupfer-(I)-oxid sowie Cer-(III)-nitrat.

Selbstverständlich können auch Gemische aus mehreren der genannten Katalysatoren eingesetzt werden.

Die erforderliche Menge des Katalysators liegt vorzugsweise im Bereich von 0,01 bis 10 mmol je Mol Mercaptothiazol bzw. Äquivalent Thiazolyl-2,2'-disulfid. Es können auch geringere Katalysatormengen zur Anwendung gelangen, jedoch müssen hierbei längere Reaktionszeiten in Kauf genommen werden. Höhere Katalysatormengen sind aus ökonomischen Gründen und wegen möglicher Verunreinigung des Reaktionsprodukts nicht mehr empfehlenswert.

Die Anwesenheit von Ammoniak als Bestandteil des Katalysatorsystems ist wesentlich; bei Abwesenheit von Ammoniak findet praktisch keine Umsetzung statt (s. Versuch V1). Die Menge des erfindungsgemäß einzusetzenden Ammoniaks kann in weiten Grenzen variiert werden. Schon ein Ammoniak-Anteil von nur 0,2 Gew.-%, bezogen auf das Gewicht des Reaktionsgemisches, zeigt einen vorteilhaften Effekt. Ein Anteil von 25 Gew.-% Ammoniak sollte nicht überschritten werden. Es ist besonders vorteilhaft, einen Anteil von 1 bis 15 Gew.-% Ammoniak, bezogen auf das Gewicht des Reaktionsgemisches, einzusetzen. Bei Ammoniak-Anteilen im oberen Bereich liegt die Reaktionsgeschwindigkeit besonders hoch, allerdings verbunden mit etwas geringerer Ausbeute.

Die Wahl des Reaktionsmediums ist für das erfindungsgemäße Verfahren von großem Einfluß und hängt insbesondere von der Natur des umzusetzenden heterocyclischen Amins ab. Sehr geeignet sind inerte wassermischbare organische Lösungsmittel, z.B. Amide wie Dimethylformamid, N-Methylpyrrolidon, Nitrile wie Acetonitrile, Ether wie Glykolalkylether, vorzugsweise niedere Alkohole, Gemische dieser Lösungsmittel oder Gemische dieser Lösungsmitteln mit Wasser. Als niedere Alkohole werden geradkettige, verzweigte oder cyclische Alkohole mit 1 bis 6 C-Atomen bevorzugt. Bei Verwendung von Lösungsmittel/Wasser-Gemischen wird ein Wasser-Anteil bis zu 50 Gew.-%, bezogen auf Gesamtgewicht des Reaktionsmediums, bevorzugt, da bei höheren Anteilen Wasser verringerte Ausbeuten zu verzeichnen sind.

In einzelnen Fällen, z.B. bei zu geringer Mischbarkeit des heterocyclischen Amins mit Alkohol bzw. Alkohol/Wasser, oder zur Erhöhung der Katalysatorlöslichkeit ist es vorteilhaft, dem Reaktionsgemisch ein weiteres, insbesondere wassermischbares Lösungsmittel zuzusetzen. Im allgemeinen wird aber bevorzugt, ohne ein zusätzliches Lösungsmittel zu arbeiten.

Verfahrenswesentlich ist auch die Reaktionstemperatur. Sie liegt erfindungsgemäß im Bereich von 0 bis 100 °C. Unterhalb dieses Bereichs ist die Reaktionsgeschwindigkeit wirtschaftlich nicht mehr interessant, während oberhalb dieses Bereichs die Selektivität stark verringert ist. Besonders bevorzugt wird das erfindungsgemäße Verfahren im Bereich von 20 bis 80 °C durchgeführt.

Die Durchführung des erfindungsgemäßen Verfahrens erfolgt in einfacher Weise dadurch, daß der Sauerstoff bzw. das sauerstoffhaltige Gas auf das Reaktionsgemisch aufgepreßt oder in bzw. durch das Reaktionsgemisch geleitet wird, das aus dem sekundären heterocyclischen Amin, Schwefel, Mercaptothiazol bzw. Dithiazolyl-2,2'-disulfid, Metallkatalysator, Ammoniak und dem Reaktionsmedium besteht.

Das Mercaptothiazol bzw. Dithiazolyl-2,2'-disulfid und/oder das sekundäre heterocyclische Amin sowie Schwefel können auch während der Reaktion dem Reaktionsgemisch zugeführt werden.

Die Reaktionsdauer hängt dabei stark von den Verfahrensbedingungen und den Reaktionspartnern ab und kann mehrere Stunden, unter günstigen Bedingungen aber auch nur wenige Minuten betragen.

In den meisten Fällen fällt das gewünschte Endprodukt bereits während der Reaktion oder am Reaktionsende nach Abkühlen in fester Form aus dem Reaktionsgemisch aus und kann abfiltriert werden. In anderen Fällen erhält man das Produkt durch Verdünnen mit Wasser oder Einengen des Reaktionsgemisches. Flüssige Produkte werden durch destillative oder extraktive Aufarbeitung in reiner Form erhalten.

Bei der technischen Durchführung des erfindungsgemäßen Verfahrens ist es vorteilhaft, die Mutterlauge im Kreislauf zu führen.

Nach dem Abfiltrieren des Endprodukts kann die Mutterlauge an 2-Mercaptothiazol bzw. Dithiazolyl-2,2'-disulfid sowie an heterocyclischem Amin und Schwefel aufgefrischt und direkt sowie praktisch beliebig oft wieder eingesetzt werden, ohne daß die Selektivität und die Ausbeute nachteilig beeinflußt werden. Das

Verfahren eignet sich daher vorzüglich für eine kontinuierliche Arbeitsweise.

Das erfindungsgemäße Verfahren erfüllt wesentliche Kriterien für eine wirtschaftliche Produktion vor allem des technisch als Vulkanisationsbeschleuniger interessanten 2-(4-Morpholinodithio)-benzthiazols: Es arbeitet gegenüber dem Stand der Technik mit einem leichter zugänglichen und handhabbaren, billigeren Oxidationsmittel bereits mit praktisch stöchiometrischen Anteilen der leicht zugänglichen Startsubstanzen bei hohem Umsatz und sehr hoher Selektivität. Ferner werden im erfindungsgemäßen Verfahren - entsprechend dem Reaktionsschema - keine Beiprodukte gebildet, also anders als bei Verfahren des Standes der Technik, gemäß welchen beispielsweise noch anorganische Verbindungen (z.B. Chloride bzw. Sulfate bei Verwendung von Hypochloriten bzw. Persulfaten als Oxidationsmittel) entstehen, die das Abwasser belasten und eine aufwendige Entsorgung erforderlich machen sowie die cyclische Prozeßführung behindern würden.

Hohe Selektivität bedeutet, daß die Produktausbeute weitgehend dem Umsatz entspricht, Materialverluste durch Nebenprodukt-Bildung also sehr gering sind. Je nach Verfahrensbedingungen enthält die Mutterlauge noch nicht umgesetztes Mercaptothiazol sowie in manchen Fällen auch Dithiazolyldisulfid. Beide können dem Verfahren erneut zugeführt werden, da beide Startverbindungen für das erfindungsgemäße Verfahren sind.

Das mit dem erfindungsgemäßen Verfahren ohne zusätzliche Reinigungsmaßnahmen erhältliche Produkt zeichnet sich durch eine besonders hohe Reinheit aus. Es werden Produkte mit einer Reinheit um 98 % und weit darüber hinaus erhalten, wobei für das 2-(4-Morpholinodithio)-benzthiazol jeweils ein Schmelzpunkt im Bereich zwischen 128° und 131° ermittelt wird. Da Produkte gemäß oben zitierter Druckschrift DE-A 22 38 516 selbst im günstigsten Fall nur bei 125° bis 127°C, im übrigen aber weit darunter schmelzen sollen (vgl. Tabelle auf Seite 7), ist auch diesbezüglich das erfindungsgemäße Verfahren demjenigen des Standes der Technik überlegen. Das handelsübliche 2-(4-Morpholinodithio)-benzthiazol schmilzt im Bereich von 123° bis 135°C, und in der Fachliteratur ist für äußerst reines 2-(4-Morpholinodithio)-benzthiazol ein Schmelzpunkt von 132-134°C angegeben. Angesichts der niedrigen Schmelzpunkte von Produkten gemäß DE-A 22 38 516 erscheinen im übrigen auch die Reinheitswerte in der Tabelle auf Seite 7 von maximal 98 % als zu hoch angegeben. Angaben darüber, nach welcher Reinheitsbestimmungsmethode diese Werte erhalten wurden, fehlen in dieser Druckschrift; ganz offensichtlich aber kamen diese Werte durch eine Schnellbestimmung zustande, bei der auch freier Schwefel und 2,2'-Dibenzthiazolyldisulfid mit erfaßt werden. Die für die erfindungsgemäß erhältlichen Produkte angegebenen Reinheitswerte resultierten hingegen aus einer Bestimmungs-Methode, welche die Miterfassung der genannten Verunreinigungen mit Sicherheit ausschließt (Titration nach J.G. Lichty; s. Beispiel 1).

Da das erfindungsgemäße Verfahren auch in Gegenwart von Wasser ausgeführt werden kann, kann das 2-Mercaptobenzthiazol als Startverbindung auch direkt in wasserfeuchtem Zustand, in dem es aus der 2-Mercaptobenzthiazol-Herstellung anfällt, zugeführt werden.

Die vorliegende Erfindung wird durch die nachfolgenden Versuchsbeispiele näher erläutert:

## Beispiel 1

In einem Druckreaktionsgefäß, das mit einem Doppelmantel zur Zirkulation einer Heizflüssigkeit, einem Thermometer, einem Druckmeßgerät und einer Rührvorrichtung ausgerüstet ist, werden 23,9 g 2-Mercaptobenzthiazol (143 mmol), 13,0 g 4-Morpholin (149 mmol), 4,6 g Schwefel (143 mmol), 0,1 mmol Cu-II-acetat, 10,7 g Ammoniak und 150 g Methanol vorgelegt. Das Reaktionsgemisch wird auf 30°C erwärmt, intensiv gerührt und mit 3 bar Sauerstoff beaufschlagt. Sofort wird eine Sauerstoffaufnahme registriert; es entsteht eine klare Lösung, anschließend fällt ein hellbeiger Feststoff aus. Nach 5 h wird die Reaktion beendet. Der Niederschlag wird abfiltriert, gewaschen und getrocknet.

So werden 35,4 g eines Produktes erhalten, das in seinen analytischen Daten (Elementaranalyse, IR, $^1$HNMR, Massenspektrometrie) mit 2-(4-Morpholinodithio)-benzthiazol identisch ist. Die Reinheit beträgt 99.5 % (Titration nach J.G. Lichty, J. Applied Chem., 2, 16, (1963)), der Schmelzpunkt 128 - 130°C. Die Mutterlauge enthält noch weitere 3,1 g des Produktes, die z.B. durch Einengen der Mutterlauge auf ca. 30 g ausfallen und abfiltriert werden können. Die Gesamtausbeute an 2-(4-Morpholinodithio)-benzthiazol beträgt demnach 38,5 g (94.8 % d.Th.).

## Beispiel 2

Es wird wie in Beispiel 1 gearbeitet, jedoch bei einem Sauerstoffdruck von $4.10^5$ Pa, und als Lösungsmittel wird ein Gemisch aus 120 g Methanol und 30 g Wasser eingesetzt. Die Reaktionszeit beträgt 220 Minuten. Die Gesamtausbeute an Morpholinodithiobenzthiazol beträgt 36,5 g (87.5 % d.Th.) bei einer Reinheit von 99.0 % (Fp. 129-131°C, MBT-Umsatz 87,8%).

**Beispiel 3**

Es wird wie in Beispiel 2 gearbeitet, jedoch mit 19,5 g 4-Morpholin (215 mmol). Die Reaktionszeit beträgt 115 Minuten. Die Gesamtausbeute an Morpholinodithiobenzthiazol beträgt 36,1 g (88.9 % d.Th.) bei einer Reinheit von 97.4 % (MBT-Umsatz 89,6%).

**Beispiel 4**

Es wird wie in Beispiel 2 gearbeitet, jedoch bei einer Reaktionstemperatur von 50 ° C, und als Lösungsmittel wird ein Gemisch aus 75 g Methanol und 75 g Wasser eingesetzt. Die Reaktionszeit beträgt 90 Minuten. Der Umsatz an Mercaptobenzthiazol liegt bei 90 %, die Ausbeute an Morpholinodithiobenzthiazol bei 85.1 % d.Th. (Fp. 128-131 ° C).

**Beispiel 5**

Es wird wie in Beispiel 2 gearbeitet, jedoch bei einer Reaktionstemperatur von 50 ° C und anstelle von 2-Mercaptobenzthiazol werden 71,5 mmol 2,2'-Dibenzthiazolyldisulfid eingesetzt. Die Reaktionszeit beträgt 41 Minuten. Die Ausbeute an Mercaptobenzthiazol beträgt 85.2 % d.Th. bei einer Reinheit von 98.0 % (Fp. 128-130 ° C).

**Beispiel 6**

Es wird wie in Beispiel 2 gearbeitet, jedoch bei einer Reaktionstemperatur von 60 ° C, und als Lösungsmittel werden 150 g Isopropanol eingesetzt. Nach 130 Minuten Reaktionszeit beträgt die Ausbeute an Marpholinodithiobenzthiazol 88.8 % d.Th. (Fp. 129-131 ° C, MBT-Umsatz 92,4%).

**Vergleichsbeispiel 1 (V1)**

Es wird wie in Beispiel 1 gearbeitet, aber ohne Zusatz von Ammoniak.

Nach 6 Stunden wird praktisch kein Sauerstoff aufgenommen. Beim Filtrieren des Reaktionsgemisches werden 3,75 g Schwefel unverändert zurückgewonnen. Die Mutterlauge enthält unverändertes Mercaptobenzthiazol.

**Vergleichsbeispiel 2 (V2)**

Es wird wie in Beispiel 6 gearbeitet, aber ohne Zusatz von Schwefel. Die sauerstoffaufnahme erfolgt wesentlich langsamer. Nach 3.5 h Reaktionszeit wird lediglich Morpholinothiobenzthiazol in einer Ausbeute von 37.4 % d.Th. erhalten. Dieses Beispiel zeigt, daß das erfindungsgemäße Verfahren auf einem neuen Reaktionsprinzip beruht und nicht durch oxidative Kupplung von Mercaptobenzthiazol und Morpholin und anschließender Reaktion des so gebildeten Sulfenamids mit Schwefel zum Disulfid erklärt werden kann.

**Beispiel 7**

Es werden 23,9 g Mercaptobenzothiazol (143 mmol), 215 g N-Methylpiperazin (215 mmol), 4,6 g Schwefel (143 mmol), 0,1 mmol Cu-II-acetat und 10 g Ammoniak in 75 g Wasser und 75 g Methanol mit Sauerstoff ($3.10^5$ Pa) wie in Beispiel 1 beschrieben zur Reaktion gebracht. Die Reaktionstemperatur beträgt 40 ° C, die Reaktionszeit 3.5 h.

Der gebildete hellbeige Feststoff wird abfiltiert, gewaschen und getrocknet und ist gemäß den analytischen Daten (Fp. 108 - 110 ° C, Elementaranalyse, $^1$HNMR, IR) identisch mit N-Methyl-piperazyl-dithiobenzthiazol. Die Ausbeute beträgt 35.7 g (83.8 % d.Th.). Die Mutterlauge enthält noch 9.9% nicht umgesetztes MBT sowie 4.4% Dibenzothiazolyldisulfid.

**Beispiel 8**

Es werden 143 mmol Mercaptobenzothiazol, 149 mmol Piperidin, 143 mmol Schwefel, 0,1 mmol Cu-II-acetat und 40 g 25%ige wäßrige Ammoniaklösung in 120 g Methanol mit Sauerstoff ($4.10^5$ Pa) wie in Beispiel 1 beschrieben zur Reaktion gebracht. Die Reaktionstemperatur beträgt 52 ° C, die Reaktionszeit 118 Minuten. Nach Abkühlen auf Raumtemperatur wird der Feststoff abfiltriert, gewaschen und getrocknet.

Dabei werden 37,4 g eines hellen Feststoffs erhalten, der in seinen analytischen Daten (IR, Elementaranalyse, Fp. 83 °C) Piperidinodithiobenzothiazol entspricht. Die Ausbeute beträgt 36,4 g (92.7 % d.Th.; MBT-Umsatz 96.1%).

**Beispiel 9**

Es werden 143 mmol MBT, 149 mmol Hexamethylenimin, 143 mmol Schwefel, 0,5 mmol Cu-II-acetat und 10 g Ammoniak in 150 g Methanol bei 25 °C mit Sauerstoff ($3.10^5$ Pa) zur Reaktion wie in Beispiel 1 beschrieben gebracht. Die Reaktionszeit beträgt 150 Minuten. Der gebildete Niederschlag wird abfiltiert, mit Methanol gewaschen und getrocknet. Dabei werden 36,9 g (90.5 % d.Th.) Hexamethyleniminodithiobenzthiazol (beige farbener Feststoff), nachgewiesen durch Elementaranalyse, $^1$H-NMR, IR und Fp. (65 - 66 °C), erhalten.

**Beispiele 10 bis 13**

143 mmol Mercaptobenzothiazol, 149 mmol Morpholin, 143 mmol Schwefel und 40 g 25%ige wäßrige Ammoniaklösung werden in 120 g Methanol in Gegenwart von 0.5 mmol verschiedener Katalysatoren in der in Beispiel 1 angegebenen Weise bei einer Temperatur um 50 °C mit Sauerstoff ($4 \cdot 10^5$ Pa) zur Reaktion gebracht. Jeweiliger Katalysator, Reaktionszeit und Produktausbeute sind der folgenden Zusammenstellung zu entnehmen:

| Beispiel spiel | Katalysator | R-Zeit (min) | Ausbeute (% d.Th.) | MBT-Umsatz (%) |
|---|---|---|---|---|
| 10 | CuCl | 25 | 81.3 | 84.1 |
| 11 | $Cu_2O$ | 11 | 83.0 | 87.4 |
| 12 | Cu (Pulver) | 24 | 86.4 | 90.8 |
| 13 | $Ce(NO_3)_3$ | 121 | 80.8 | 84.7 |

**Beispiel 14**

Es wird wie in Beispiel 2 gearbeitet, jedoch mit 0.05 mmol Cu-II-acetat und bei einer Temperatur von 50 °C. Die Reaktionszeit beträgt 138 Minuten und die Produktausbeute 82.3 % der Theorie (Fp. 130-132 °C; MBT-Umsatz 84.1%).

**Beispiele 15 und 16**

Es wird wie in Beispiel 2 gearbeitet, jedoch mit 21,4 g Ammoniak (Beispiel 15) und 5,4 g Ammoniak (Beispiel 16) bei einer Temperatur von 50 °C. Ferner kommen in Beispiel 16 nicht 0.1 mmol, sondern 0.2 mmol Cu-II-acetat zum Einsatz. Die Reaktionszeiten betragen 50 min (Beispiel 15) und 4 Stunden (Beispiel 16) und die Produktausbeuten 81.4 % (MBT-Umsatz 84.5%, Beispiel 15) und 86.6 % d.Th. (MBT-Umsatz 87.1%, Beispiel 16).

**Beispiel 17**

Es wird wie in Beispiel 16 gearbeitet, jedoch mit 0.5 mmol Cu-II-acetat und bei einem Sauerstoffpartialdruck von $0.6 \cdot 10^5$ Pa. Die Reaktionszeit beträgt 124 Minuten und die Produktausbeute 85.1 % d.Th. (Fp. 128-130 °C; MBT-Umsatz 87.2%).

**Beispiel 18**

Es werden 71.5 mmol Mercaptobenzthiazol, 74.5 mmol Morpholin, 71.5 mmol Schwefel, 0.25 mmol Cu-II-acetat, 20 g 25%ige wäßrige Ammoniaklösung in 60 g Methanol wie in Beispiel 1 beschrieben bei einer Temperatur von 50 °C oxidiert, jedoch wird als sauerstoffhaltiges Gas Luft eingesetzt (Druck $5 \cdot 10$ Pa). Nach einer Reaktionszeit von 161 min wird Morpholinodithiobenzthiazol in einer Ausbeute von 84.5 % d.Th. erhalten.

**Beispiel 19**

Es werden 143 mmol Mercaptobenzthiazol, 149 mmol Morpholin, 286 mmol Schwefel (9,2 g), 0.1 mmol Kupfer-I-oxid und 40 g 25%ige wäßrige Ammoniaklösung in 120 g Methanol wie in Beispiel 1 beschrieben bei einem Sauerstoffdruck von $4 \cdot 10^5$ Pa zur Reaktion gebracht. Die Reaktionstemperatur beträgt 52°C und die Reaktionszeit 43 Minuten. Der gebildete hellbeige Niederschlag wird abfiltiert, gewaschen und getrocknet. Aufgrund seiner IR-Spektren, der Elementaranalyse sowie seiner vollständigen Löslichkeit in Alkohol ist dem erhaltenen Produkt die Struktur von Morpholinotrithiobenzothiazol zuzuordnen. Das Produkt schmilzt bei 123 bis 126°C und weist eine Reinheit von 98.5 % auf (Titration nach Lichty). Die Ausbeute beträgt 40,84 g (90.2 % d.Th.).

**Beispiel 20**

Es wird wie in Beispiel 1 gearbeitet, jedoch werden 12.5 g 4-Morpholin (143 mmol) und 10 g Ammoniak eingesetzt. Die Reaktionszeit beträgt 5 h. Die Ausbeute an Morpholinodithiobenzthiazol beträgt 37.6 g (92.4 % d. Th.) bei einer Reinheit von 99.5 % (Fp. 128 - 130°C). Der Umsatz an 2-Mercaptobenzthiazol liegt bei 94 %. Daneben enthält die Mutterlauge noch 0.3 g Dibenzothiazyldisulfid, das als Zwischenprodukt angesehen werden kann.

**Beispiel 21**

Es wird wie in Beispiel 20 gearbeitet, jedoch 4-Morpholin in stöchiometrischem Unterschuß eingesetzt (11.3 g, 130 mmol). Nach 5.5 h Reaktionszeit werden 33.3 Morpholinodithiobenzthiazol erhalten, entsprechend einer Ausbeute von 90.1 % d. Th. (Reinheit 99.3 %, Fp. 128 - 130°C, das Produkt ist vollständig löslich in Methanol). Der Umsatz an 2-Mercaptobenzthiazol beträgt 95.6 %. Daneben enthält die Mutterlauge noch 3 g des Zwischenproduktes Dibenzothiazyldisulfid.

**Patentansprüche**

**1.** Verfahren zur Herstellung von 2-Aminodithiothiazolen und/oder 2-Aminotrithiothiazolen der allgemeinen Formel (I)

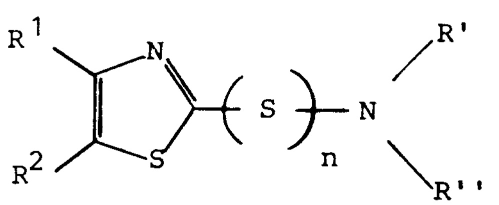

(I)

in der n im wesentlichen 2 oder 3 ist, in der $R^1$ und $R^2$ gleich oder verschieden sein können und jeweils für ein Wasserstoffatom, ein Halogenatom, eine Nitrogruppe, eine Hydroxylgruppe oder für einen gegebenenfalls ein- oder mehrfach substituierten organischen Rest wie einen Alkyl- oder Alkoxylrest mit 1 bis 6 Kohlenstoffatomen oder einen Cycloalkyl- oder Arylrest mit 6 bis 12 Kohlenstoffatomen stehen, wobei die Substituenten jeweils ein Halogenatom, eine Nitrogruppe, eine Hydroxylgruppe oder einen Alkyl- oder Alkoxyrest mit 1 bis 5 Kohlenstoffatomen sein können, oder in der $R^1$ und $R^2$ gemeinsam den Rest (II)

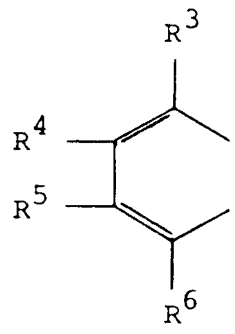

oder

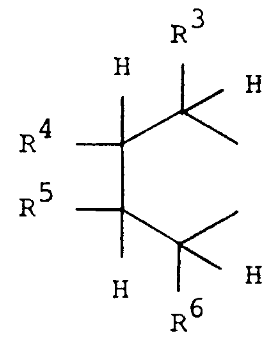

(II)

bilden, wobei $R^3$, $R^4$, $R^5$ und $R^6$ gleich oder verschieden sein können und jeweils dieselbe Bedeutung haben wie $R^1$ und $R^2$, nicht jedoch den Rest (II) bilden, und in der R' und R'' zusammen mit dem Aminstickstoff einen aliphatisch gesättigten heterocyclischen Ring bilden, der mindestens ein weiteres Heteroatom enthalten kann, wobei mit Piperazin als Basis des heterocyclischen Rings das weitere Stickstoffatom ebenfalls den 2-Thiazolyldithio- bzw. 2-Thiazolyltrithio-Rest tragen kann, durch Umsetzung einer Mischung eines 2-Mercaptothiazols der allgemeinen Formel (III) oder Dithiazolyl-2,2'-disulfids der allgemeinen Formel (IV)

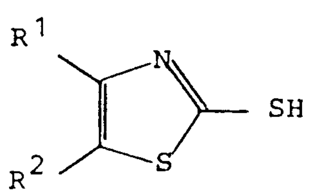

(III)

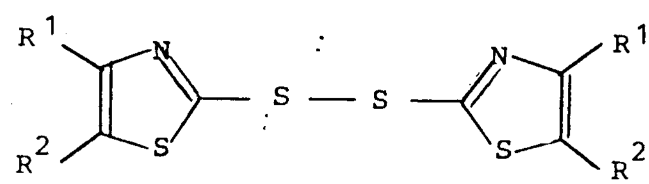

(IV)

in der $R^1$ und $R^2$ die oben angegebene Bedeutung haben, eines gesättigten sekundären, heterocyclischen Amins der allgemeinen Formel (V)

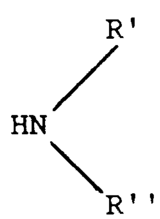

(V)

mit der oben angegebenen Bedeutung für R' und R'' und Schwefel in einem ein inertes organisches Lösungsmittel enthaltenden Reaktionsmedium in Gegenwart eines Oxidationsmittels, dadurch gekennzeichnet, daß die Umsetzung in Gegenwart von Ammoniak und eines Kupfer, eine Kupferverbindung oder eine Cer-Verbindung enthaltenden Katalysators durchgeführt wird und daß das Oxidationsmittel molekularer Sauerstoff oder ein diesen Sauerstoff enthaltendes Gas ist.

**2.** Verfahren gemäß Anspruch 1, dadurch gekennzeichnet, daß die Substituenten $R^1$ und $R^2$ die Reste der allgemeinen Formel (II) bilden und/oder die Substituenten $R^1$ bis $R^6$ ein Chlor- oder Bromatom, eine Hydroxylgruppe, eine Nitrogruppe, ein geradkettiger oder verzweigter Alkylrest mit 1 - 4 Kohlenstoffatomen wie Methyl-, Ethyl-, Propyl-, Isopropyl-, Butyl- oder tert.-Butylrest, ein Alkoxyrest mit 1 - 4 Kohlenstoffatomen wie Methoxy-, Ethoxy-, Propoxy- oder Butoxy- oder ein Phenyl-, Tolyl-, Ethylphenyl-, Nitrophenyl-, Chlorphenyl-, Bromphenyl- oder Naphthylrest sind.

3. Verfahren gemäß Anspruch 1 oder 2, dadurch gekennzeichnet, daß das sekundäre heterocyclische Amin der allgemeinen Formel (V) unsubstituiertes oder mit Alkylgruppen mit 1 bis 4 C-Atomen substituiertes Pyrrolidin, Pyrazolidin, Imidazolidin, Oxazolidin, Thiazolidin, Cyclohexamethylenimin und besonders bevorzugt unsubstituiertes oder substituiertes Piperidin, Piperazin und 4-Morpholin ist.

4. Verfahren gemäß einem oder mehreren der Ansprüche 1 bis 3, dadurch gekennzeichnet, daß 0.9 bis 2 Mol und bevorzugt 0.9 bis 1.5 Mol Startverbindung (V) je Mol Startverbindung (III) bzw. Äquivalent Startverbindung (IV), eingesetzt werden.

5. Verfahren gemäß einem oder mehreren der Ansprüche 1 bis 4, dadurch gekennzeichnet, daß als Katalysator Kupferpulver, Kupfer-(I)-chlorid, Kupfer-(III)-acetat, Kupfer-(II)-sulfat, Kupfer-(II)-oleat, Kupfer-(II)-acetylacetonat, Kupfer-(II)-sulfid, Kupfer-(I)-oxid und/oder Cer-(III)-nitrat, eingesetzt wird.

6. Verfahren gemäß einem oder mehreren der Ansprüche 1 bis 5, dadurch gekennzeichnet, daß der Ammoniak in Mengen von 0,2 bis 25 Gew.-% und bevorzugt von 1 bis 15 Gew.-%, bezogen auf das Gewicht des Reaktionsgemisches, eingesetzt wird.

7. Verfahren gemäß einem oder mehreren der Ansprüche 1 bis 6, dadurch gekennzeichnet, daß als Dispersionsmedium inerte wassermischbare organische Lösungsmittel oder deren Gemische mit Wasser eingesetzt werden.

8. Verfahren gemäß Anspruche 7, dadurch gekennzeichnet, daß das Reaktionsmedium aus gesättigten Alkoholen mit 1 bis 6 C-Atomen oder Gemischen dieser Lösungsmittel mit Wasser, bevorzugt bis zu 50 Gew.-% Wasser, bezogen auf das Gesamtgewicht des Reaktionsmediums, besteht.

9. Verfahren gemäß einem oder mehreren der Ansprüche 1 bis 8, dadurch gekennzeichnet, daß die Umsetzung bei Temperaturen im Bereich zwischen 0 und 100 ° C und bevorzugt im Bereich zwischen 20 und 80 ° C durchgeführt wird.

Europäisches Patentamt

# EUROPÄISCHER RECHERCHENBERICHT

Nummer der Anmeldung

EP 91 11 7334

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | DE-A-2 164 480 (BAYER AKTIENGESELLSCHAFT)<br>* Ansprüche * * | 1 | C 07 D 277/78<br>C 07 D 277/36 |
| A | EP-A-0 001 098 (BAYER AKTIENGESELLSCHAFT)<br>* Ansprüche * * | 1 | |
| A | FR-A-2 503 158 (AKZO NV)<br>* Ansprüche * * | 1-9 | |

| RECHERCHIERTE SACHGEBIETE (Int. Cl.5) |
|---|
| C 07 D |

Der vorliegende Recherchenbericht wurde für alle Patentansprüche erstellt

| Recherchenort | Abschlußdatum der Recherche | Prüfer |
|---|---|---|
| Den Haag | 07 Januar 92 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus anderen Gründen angeführtes Dokument

&: Mitglied der gleichen Patentfamilie, übereinstimmendes Dokument